(19) Europäisches Patentamt
European Patent Office
Office européen des brevets

(11) Publication number : **0 232 067 B2**

(12) **NEW EUROPEAN PATENT SPECIFICATION**

(45) Date of publication of the new patent specification : **16.03.94 Bulletin 94/11**

(21) Application number : **87300502.9**

(22) Date of filing : **21.01.87**

(51) Int. Cl.$^5$ : **C07D 401/12,** A01N 47/36, C07D 213/81, C07D 213/82, C07D 213/89, // C07D213/80

# (54) Substituted pyridinesulfonamide compounds, herbicidal composition containing them, and method of preparing these compounds.

The file contains technical information submitted after the application was filed and not included in this specification

(30) Priority : **30.01.86 JP 19006/86**
**31.01.86 JP 19863/86**
**15.04.86 JP 86847/86**
**29.07.86 JP 178489/86**

(43) Date of publication of application :
**12.08.87 Bulletin 87/33**

(45) Publication of the grant of the patent :
**06.03.91 Bulletin 91/10**

(45) Mention of the opposition decision :
**16.03.94 Bulletin 94/11**

(84) Designated Contracting States :
**AT CH DE ES FR GB IT LI NL**

(56) References cited :
**EP-A- 0 007 687**
**EP-A- 0 013 480**
**EP-A- 0 030 138**
**EP-A- 0 035 893**
**EP-A- 0 101 407**
**EP-A- 0 101 670**
**EP-A- 0 237 292**
**Modern Corn Production. Samuel R Aldrich et al., 1986, pages 219-235**
**Farmers Weed Control Handbook, 1985, pages 21-41**
**Plantes Herbicides et Desherbicides. M Tissut and F Severin. 1984, pages 110 and 193**
**Le desherbage du mais-quel produit pour quelle mauvaise herbe ? 1989. Association Generale des Producteurs de mais**

(73) Proprietor : **ISHIHARA SANGYO KAISHA LTD.**
**No. 3-11, Edobori 1-chome**
**Nishi-ku Osaka-shi Osaka 550 (JP)**

(72) Inventor : **Kimura, Fumio, c/o Ishihara Sangyo K.K.**
**Chuo Kenkyusho, 3-1, Nishishibukawa 2-chome**
**Kusatsu-shi, Shiga-ken (JP)**
Inventor : **Haga, Takahiro, c/o Ishihara Sangyo K.K.**
**Chuo Kenkyusho, 3-1, Nishishibukawa 2-chome**
**Kusatsu-shi, Shiga-ken (JP)**
Inventor : **Sakashita, Nobuyuki, c/o Ishihara Sangyo K.K.**
**Chuo Kenkyusho, 3-1, Nishishibukawa 2-chome**
**Kusatsu-shi, Shiga-ken (JP)**
Inventor : **Honda, Chimoto, c/o Ishihara Sangyo K.K.**
**Chuo Kenkyusho, 3-1, Nishishibukawa 2-chome**
**Kusatsu-shi, Shiga-ken (JP)**
Inventor : **Murai, Shigeo, c/o Ishihara Sangyo K.K.**
**Chuo Kenkyusho, 3-1, Nishishibukawa 2-chome**
**Kusatsu-shi, Shiga-ken (JP)**

(74) Representative : **Pearce, Anthony Richmond et al**
**MARKS & CLERK Alpha Tower Suffolk Street**
**Queensway Birmingham B1 1TT (GB)**

EP 0 232 067 B2

## Description

The present invention relates to a novel substituted pyridinesulfonamide compound and its salts, a herbicidal composition containing same as an active ingredient, and a process for producing such compound and its salts.

A substituted pyridinesulfonamide compound of this invention has a pyridine ring with an N-substituted aminocarbonyl group. Such substituted pyridinesulfonamide compound is included in general formulae in US-A-4,518,776, EP-A-101407, EP-A-101670, and US-A-4,521,597. However, the substituted pyridinesulfonamide of this invention is not concretely disclosed in the above prior patents. A certain substituted pyridinesulfonamide compound is described in US-A-4,435,206. However, this compound is not one wherein the pyridine ring has an N-substituted aminocarbonyl group. EP-A-007687 and EP-A-0030138 disclose substituted phenylsulfonamides where the benzene ring has an N-substituted aminocarbonyl group. A large number of analogues of a herbicide containing a sulfonamide compound as an active ingredient have been developed. However, no herbicides showing a high safety against corns and having a high herbicidal effect have been developed yet.

The present inventors have made extensive studies on the relation between chemical structures and physiological activities against plants with respect to the sulfonamide compounds. In particular, the present inventors have made further studies for finding a herbicide for corns.

The present invention provides a substituted pyridinesulfonamide compound, its salts, a herbicidal composition containing the same, and a process for producing such compound and its salts. The substituted pyridinesulfonamide compound is represented by the following formula:

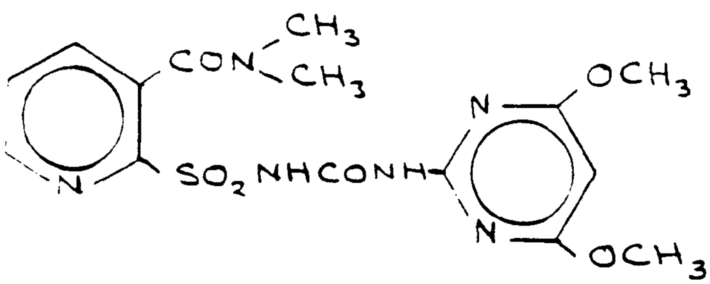

(I)

, or a salt thereof.

Salts of the substituted pyridinesulfonamide compound according to this invention include salts of alkali metals (e.g., sodium and potassium), salts of alkali earth metals (e.g., magnesium and calcium), and salts of amines (e.g., monomethylamine, dimethylamine and triethylamine). These salts may be prepared by a conventional method.

The substituted pyridinesulfonamide compound or a salt thereof according to the present invention can be prepared by the following methods:

A pyridine compound represented by the general formula

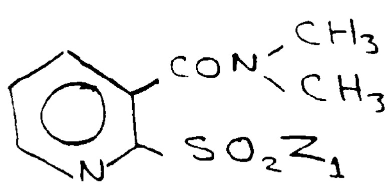

... (II)

wherein $Z_1$ represents an $-NH_2$ group, an -NCO group, an -NHCOC$\ell$ group, or an $-NHCOOR_5$ group (wherein $R_5$ represents an alkyl or aryl group) is reacted with a pyrimidine compound represented by the following general formula:

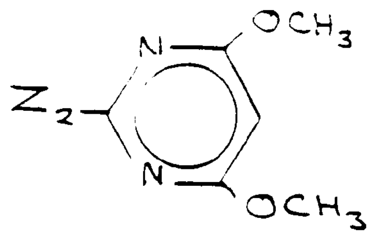

(III)

$Z_2$ represents an $NH_2$ group, an -NCO group, an -NHCOCℓ group, or a -$NHCOOR_5$ group (wherein $R_5$ represents the same as described above), provided that when $Z_1$ represents an $NH_2$ group, $Z_2$ represents an -NCO, -NHCOCℓ, or -$NHCOOR_5$ group, and that when $Z_2$ represents an $NH_2$ group, $Z_1$ represents an -NCO, -NHCOCℓ, or -$NHCOOR_5$ group. In addition, a salt formation treatment is performed, if desired.

More specifically, the pyridinesulfonamide compound and its derivative can be prepared by methods [A] to [F].

[A] 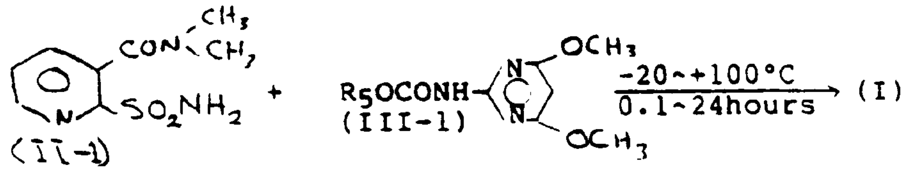


[B] 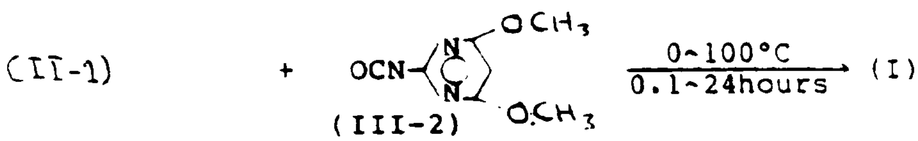


[C] 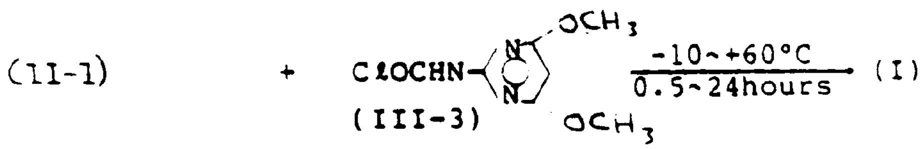


[D] 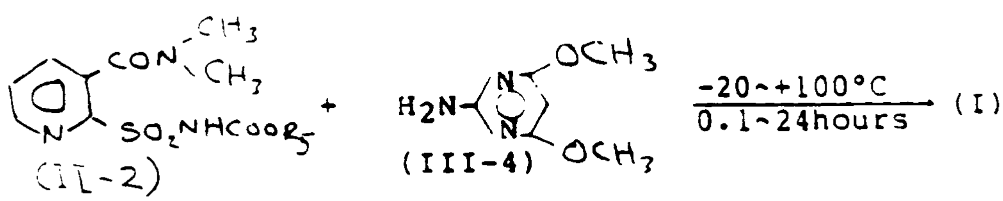


[E] 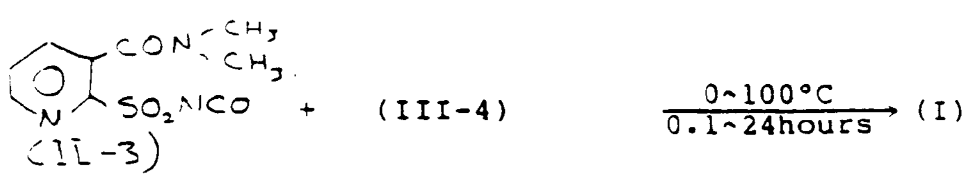


[F] 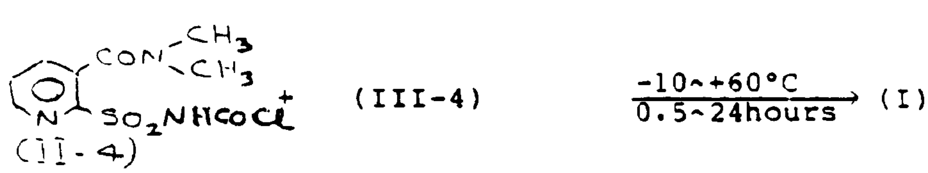

The aryl group represented by $R_5$ in formulae (III-1) and (II-2) may be a phenyl or naphthyl group which may be substituted with one or more chlorine atom or one or more methyl groups. In methods [A] and [D], 1,8-diazabicyclo[5•4•0]-7-undecene may be added to accelerate the reactions, if desired. In methods [B], [C], [E], and [F], 1,4-diazabicyclo[2•2•2]-octane may be added, if desired. In methods [B], [C], [E], and [F], a base such as triethylamine or pyridine may be added, if desired. Methods [A] to [F] are practised in the presence of a solvent, if desired. Examples of the solvent are: aromatic hydrocarbons (e.g., benzene, toluene, xylene, and chlorobenzene); cyclic or acyclic aliphatic hydrocarbons (e.g., chloroform, carbon tetrachloride, methylene chloride, dichloroethane, trichloroethane, hexane, and cyclohexane); ethers (e.g., diethylether, dioxane, and tetrahydrofuran); nitriles (e.g., acetonitrile, propionitrile, or acrylonitrile); and aprotic polar solvents (e.g., dimethylsulfoxide and sulfolane).

(1) It is shown that the pyridine compound (II-1) as the starting material for the reaction formula can be prepared by the process in routes 1 and 2:

Route 1

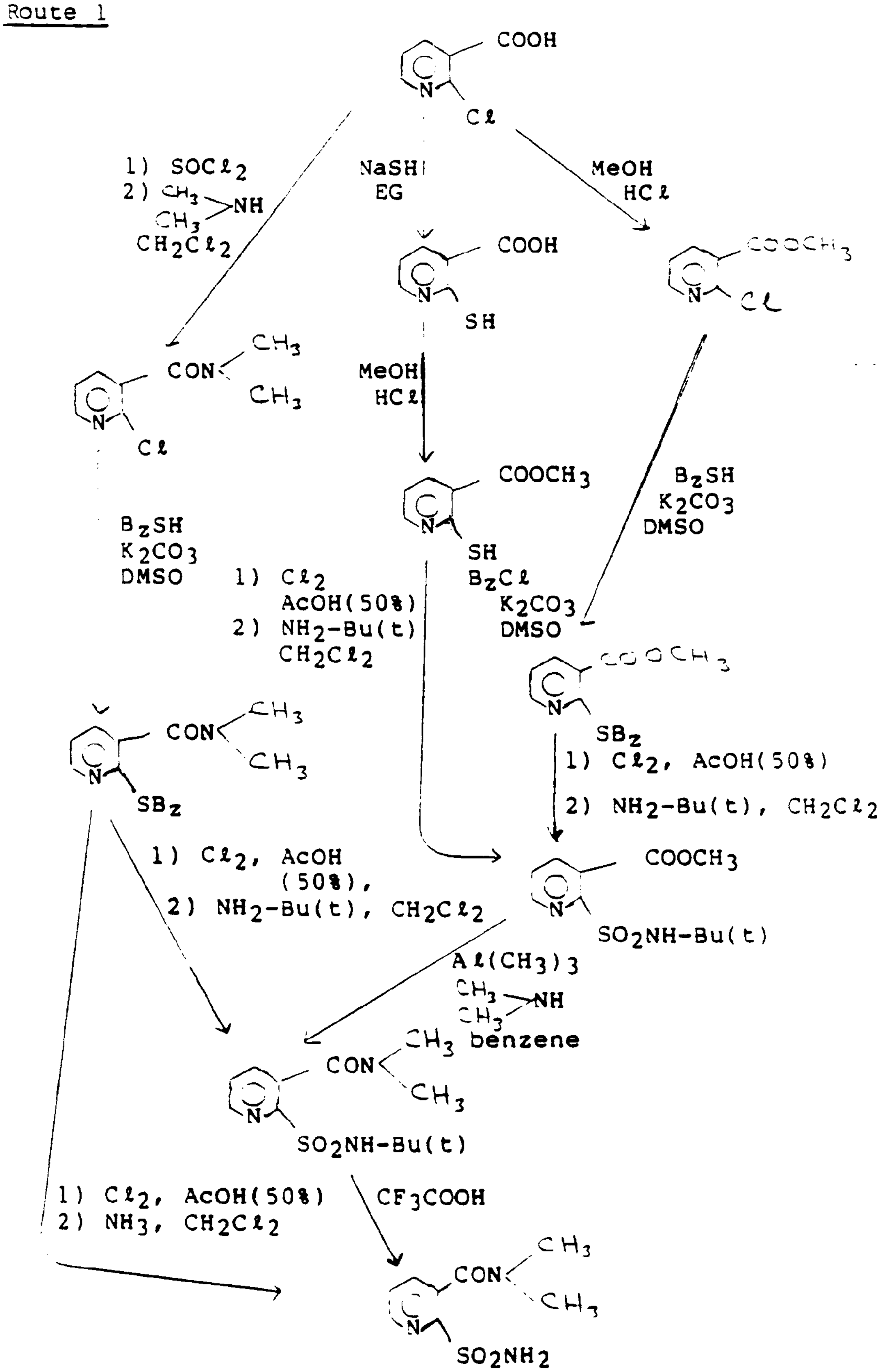
1) SOCℓ2
2) CH3>NH CH3
CH2Cℓ2
NaSH
EG
MeOH
HCℓ
MeOH
HCℓ
BzSH
K2CO3
DMSO
BzSH
K2CO3
DMSO
1) Cℓ2
AcOH(50%)
2) NH2-Bu(t)
CH2Cℓ2
BzCℓ
K2CO3
DMSO
1) Cℓ2, AcOH(50%)
2) NH2-Bu(t), CH2Cℓ2
1) Cℓ2, AcOH (50%),
2) NH2-Bu(t), CH2Cℓ2
Aℓ(CH3)3
CH3>NH CH3
benzene
1) Cℓ2, AcOH(50%)
2) NH3, CH2Cℓ2
CF3COOH

(II-1 )

Route 2

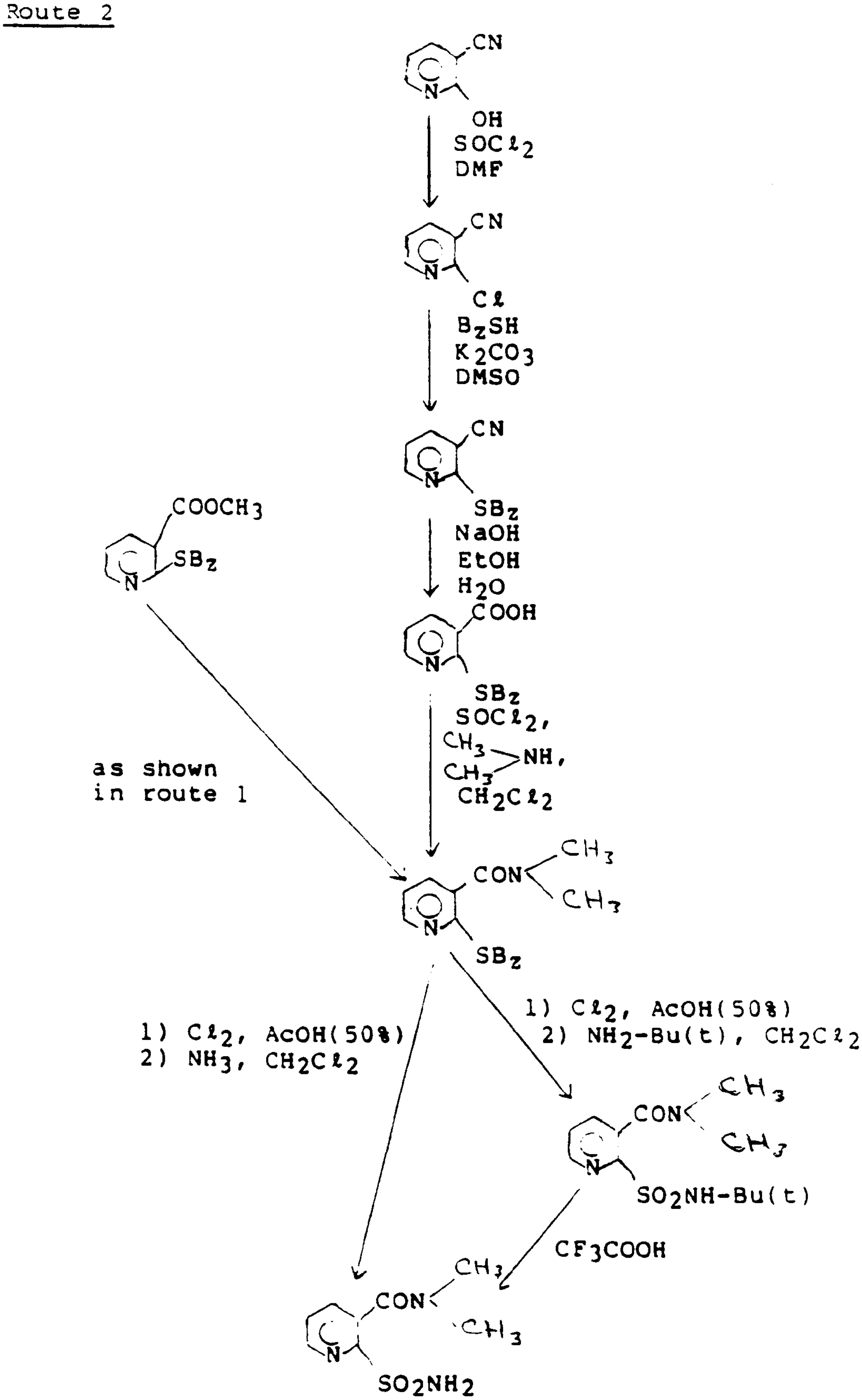


(II-1)

Note: EG is ethylene glycol, MeOH is methanol, EtOH is ethanol DMF is dimethylformamide, DMSO is

dimethylsulfoxide, BZ is a benzyl group, -Bu(t) is a tertiary butyl group, and AcOH is acetic acid.

(2) It is shown that compounds represented by the formulae (II-2) to (II-4) can be prepared from the compound of formula (II-1) by the process in route 3 below.

Route 3

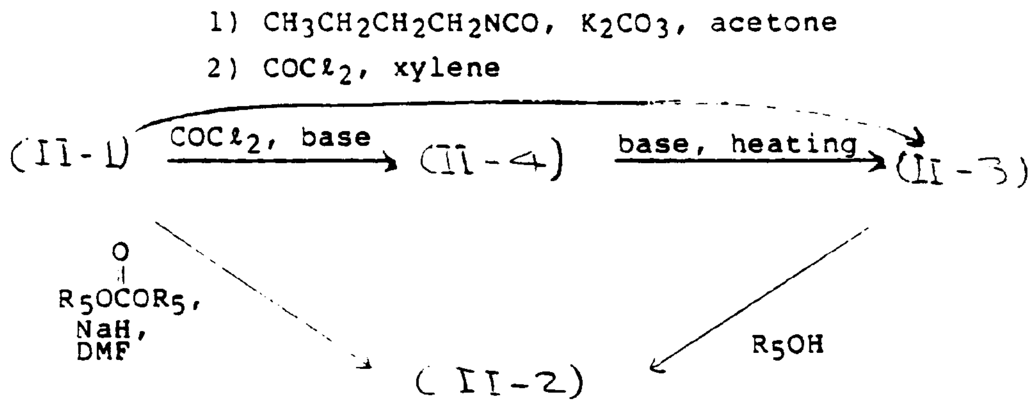


The reaction conditions for preparing the starting compound, such as reaction temperature, reaction time, a solvent which is optionally used, an alkaline substance, etc., can be suitably selected from those of the conventional analogous reactions.

Intermediate Synthesis Reference Example -Synthesis of 2-aminosulfonyl-5-chloro-N,N-dimethyl-nicotinamide

[I] 6.5 g of 2,5-dichloronicotinic acid was mixed in 24.7 mℓ of thionyl chloride and reacted therewith under the reflux condition for 2 hours. After the reaction, excessive thionyl chloride was distilled and removed, and 39 mℓ of methylene chloride was added to the mixture and 2.77 g of dimethylamine hydrochloride salt was further added thereto. 8.60 g of triethylamine was dropped within about an hour, and the resultant mixture was allowed to react at room temperature for about an hour.

After the completion of the reaction, the reaction product was pourred into water for methylene chloride extraction. A methylene chloride layer was dried with anhydrous sodium sulfate, and methylene chloride was distilled off at reduced pressure. The resultant residue was purified by a silica gel column chromatography, thereby preparing 5.5 g of 2,5-dichloro-N,N-dimethylnicotinamide having a melting point of 120 to 122°C.

[II] A mixture of 3.0 g of 2,5-dichloro-N,N-dimethylnicotinamide prepared in process [I], 1.70 g of benzylmercaptane, and 5 mℓ of dimethylsulfoxide was added dropwise in a suspension of 1.89 g of anhydrous potassium carbonate in 28 mℓ of dimethylsulfoxide at 80°C for about an hour. The resultant mixture was reacted at 130 to 140°C for 30 minutes.

After the completion of the reaction, the reaction product was added to water for methylene chloride extraction. A methylene chloride layer was dried with anhydrous sodium sulfate, and methylene chloride was distilled off at reduced pressure. The resultant residue was purified by a silica gel column chromatography to prepare 2.18 g of oily 2-benzylthio-5-chloro-N,N-dimethylnicotineamide.

[III] Chlorine gas was introduced to 20 mℓ of a 50% acetic acid solution containing 2.36 g of 2-benzylthio-5-chloro-N,N-dimethylnicotinamide at 0 to 5°C. The reaction was interrupted when excessive chlorine appeared.

After the completion of the reaction, the reaction product was added to 150 g of ice and 200 mℓ of methylene chloride to separate a methylene chloride layer. The layer was washed with 300 mℓ of ice and water to cool the layer to 0°C. Subsequently, tert-butylamine was dropped and the mixture stirred until the temperature of the layer came to room temperature. The reaction solution was checked to be weakly alkaline and the reaction was finished.

After the completion of the reaction, the reaction product was added to water for methylene chloride extraction. The extracted methylene chloride was dried with anhydrous sodium sulfate, and methylene chloride was distilled off. The resultant residue was purified by a silica gel column chromatography to prepare 1.21 g of 2-tert-butylaminosulfonyl-5-chloro-N,N-dimethylnicotinamide having a melting point of 143

to 145°C.

[IV] 1.0 g of 2-tert-butylaminosulfonyl-5-chloro-N,N-dimethylnicotinamide prepared in process [III] was added to 10 mℓ of trifluoroacetic acid and reacted therewith under the reflux condition for about an hour.

After the completion of the reaction, trifluoroacetic acid was distilled from the reaction product at reduced pressure. The residue was then purified by a silica gel column chromatography to prepare 0.71 g of 2-aminosulfonyl-5-chloro-N,N-dimethylnicotinamide having a melting point of 155 to 157°C.

Intermediate Synthesis Example -Synthesis of 2-aminosulfonyl-N,N-dimethylnicotinamide

[Method A]

[I] In a nitrogen atmosphere at 10°C or less, 10 mℓ of normal hexane containing 19% trimethylaluminum was added to a solution prepared by dissolving 1.3 mℓ of dimethylamine in 10 mℓ of anhydrous benzene. The resultant mixture was reacted until its temperature reached room temperature. A solution prepared by dissolving methyl 2-tert-butylaminosulfonylnicotinate in 40 mℓ of benzene and 20 mℓ of methylene chloride was dropped in the reacted solution. Subsequently, the resultant mixture was stirred under the reflux condition for about 9 hours.

After the completion of the reaction, the reacted solution was introduced to a dilute hydrochloric acid solution to extract with methylene chloride. The extracted substance was dried with anhydrous sodium sulfate, methylene chloride was distilled off at reduced pressure, and the residue was purified by a silica gel column chromatography to prepare 1.0 g of 2-tert-butylaminosulfonyl-N,N-dimethylnicotinamide.

[II] By using the above product and following the same procedures as in process [IV] of the Intermediate Synthesis Reference Example, 2-aminosulfonyl-N,N-dimethylnicotinamide having a melting point of 209 to 211°C was prepared.

[Method B]

Chlorine gas was introduced to 50 mℓ of 50% acetic acid solution of 2.7 g of 2-benzylthio-N,N-dimethylnicotinamide until excessive chlorine was appeared.

After the completion of the reaction, the reacted mixture was added to 200 mℓ of ice and water to extract with 60 mℓ of methylene chloride. Subsequently, a methylene chloride layer was washed with 100 mℓ of ice and water and cooled to 0°C. Ammonia gas was introduced to the solution until it was converted into an alkaline solution. After methylene chloride was distilled off, 15 mℓ of water were added to the residue, and the resultant mixture was stirred. The resultant crystal was filtered out and washed with a small amount of water. The crystal was then dried at reduced pressure to prepare 1.27 g of 2-aminosulfonyl-N,N-dimethyl-nicotinamide having a melting point of 209 to 211°C.

Synthesis Example No. 1 Synthesis of N-[(4,6-dimethoxypyrimidin-2-yl)aminocarbonyl]-3-dimethylamino carbonyl-2-pyridinesulfonamide

0.152 g of 1,8-diazabicyclo[5•4•0]-7-undecene was added to a suspension prepared by adding 0.229 g of 2-aminosulfonyl-N,N-dimethylnicotinamide and 0.275 g of 2-phenoxycarbonylamino-4,6-dimethoxypyrimidine to 5 mℓ of anhydrous acetonitrile, and the resultant mixture was reacted at room temperature for one hour.

After the completion of the reaction, the reacted mixture was added to water to filter an insoluble substance. The filtered solution was converted by concentrated hydrochloric acid to a weakly acidic solution and was extracted with methylene chloride. Subsequently, the solution was dried with anhydrous sodium sulfate, and the solvent was distilled off, thereby obtaining 0.330 g of the desired compound having a melting point of 169 ~ 173°C.

Synthesis Example No. 2 Synthesis of N-[(4,6-dimethoxypyrimidin-2-yl)aminocarbonyl]-3-dimethylaminocarbonyl-2-pyridinesulfonamide

A mixture solution of 250 mg of 2-amino-4,6-dimethoxypyrimidine, 0.65 g of triethylamine, and 2.5 g of ethyl acetate was dropped in 6.3 g of an ethyl acetate solution containing 20% of phosgene at 15°C, and the mixture was maintained at 15°C and reacted for one hour. Subsequently, the mixture was heated to 90°C on the oil bath, and excessive phosgene and ethyl acetate were distilled off. Then, a solution prepared by dissolving 300 mg of 2-aminosulfonyl-N,N-dimethylnicotinamide in 10 mℓ of acetonitrile was dropped in the above mixture, and 0.2 g of triethylamine was further dropped therein. The resultant mixture was reacted at room temperature

for one hour.

After the completion of the reaction, the product was added to water and converted by hydrochloric acid into an acidic solution, and the precipitated crystal was filtered. The crystal was washed with water and dried to obtain 0.46 g of the desired compound.

The sodium salt of N-[(4,6-dimethoxypyrimidin-2-yl)-aminocarbonyl]-3-dimethylaminocarbonyl-2-pyridinesulfonamide decomposes at 195 ~ 215°C, whilst the monomethylamine salt thereof has a melting point of 125 ~ 128°C.

The compounds according to the present invention have herbicidal effects against a variety of weeds: cyperaceous weeds ( Cyperaceae ) such as rice flatsedge ( Cyperus iria ), japanese bulrush ( Scirpus juncoides ), and purple nutsedge ( Cyperus rotundus ); gramineous weeds ( Gramineae ) such as barnyard grass ( Echinochloa crus-galli ), crab-grass ( Digitaria adscendens ), green foxtail ( Setaria viridis ), goosegrass ( Eleusine indica ), wild oat ( Avena fatua ), johnsongrass ( Sorghum halepense ), and quackgrass ( Agropyron repens ); and broadleaved weeds such as velvetleaf ( Abutilon theophrasti ), tall morningglory ( Ipomoea purpurea ), common lambsquarters ( Chenopodium album ), prickly sida ( Sida spinosa ), common purslane ( Portulaca oleracea ), slender amaranth ( Amaranthus viridis ), sicklepod ( Cassia tora ), black nightshade ( Solanum nigrum ), smartweed ( Polygonum longisetum ), common chickweed ( Stellaria media ), common cocklebur ( Xanthium strumarium ), and wavy bittercress ( Cardamine flexuosa ). Therefore, herbicides according to the present invention will find an application such as upland farms and many other applications such as agricultural fields, e.g., orchards and mulberry fields and nonagricultural fields, e.g., forests, farm roads, playgrounds, factory sites, and turf fields. The herbicidal composition of the present invention can be applied by soil treatment or foliar treatment, if desired.

In particular, the compounds according to the present invention can have herbicidal effects against noxious weeds in corns and can be effectively used. The herbicidal compound of the present invention is applied in the form of granules, wettable powder, emulsifiable concentrate or aqueous solution prepared by mixing the compound with a carrier as well as additives such as a diluent, solvent, emulsifier, spreader or surfactant, as required. A suitable mixing ratio of the active ingredient to the agricultural adjuvant(s) ranges from 1 : 99 to 90 : 10 and preferably from 5 : 95 to 60 : 40 by weight. An optimal amount of the active ingredient applied cannot be unequivocally defined because it varies according to various factors such as the climate condition, the weather condition, the soil condition, the form of the chemical, the type of weeds to be controlled, or the time of application, but the amount of the active ingredient is usually from 0.1 to 100 g per are, preferably from 0.2 to 50 g, and more preferably 0.5 g to 10 g.

The herbicidal composition of the present invention can be mixed or used together with other agricultural chemicals, fertilizers, soil, or safeners. These use brings about a more excellent effect or action. Examples of other herbicides which can be mixed with the herbicidal composition of the present invention are listed below. In some cases, synergism may be achieved.

3,6-dichloro-2-methoxybenzoic acid
2,5-dichloro-3-aminobenzoic acid
(2,4-dichlorophenoxy)acetic acid
(4-chloro-2-methylphenoxy)acetic acid
2-chloro-4,6-bis(ethylamino)-1,3,5-triazine
2-chloro-4-ethylamino-6-isopropylamino-1,3,5-triazine
2-(4-chloro-6-ethylamino-1,3,5-triazin-2-ylamino)-2-methylpropionitrile
2-ethylamino-4-isopropylamino-6-methythio-1,3,5,-triazine
2-chloro-2',6'-diethyl-N-(methoxymethyl)acetanilide
2-chloro-6'-ethyl-N-(2-methoxy-1-methylethyl)acetoo-toluidide
2-chloro-N-isopropylacetanilide
2-chloro-N,N-di-2-propenylacetamide
S-ethyl dipropylthiocarbamate
S-ethyl di-isobutylthiocarbamate
S-propyl dipropylthiocarbamate
N-(1-ethylpropyl)-2,6-dinitro-3,4-xylidine
$\alpha,\alpha,\alpha$-trifluoro-2,6-dinitro-N,N-dipropyl-p-toluidine
2-(3,5-dichlorophenyl)-2-(2,2,2-trichloroethyl) oxirane
3-isopropyl-(IH)-benzo-2,1,3-thiadiazin-4-one-2,2-dioxide
3-(3,4-dichlorophenyl)-1-methoxy-1-methylurea
3,5-dibromo-4-hydroxybenzonitrile
2-chloro-4-trifluoromethylphenyl-3-ethoxy-4-nitrophenylether

For example, N-[(4,6-dimethoxypyrimidin-2-yl)aminocarbonyl]-3-dimethylaminocarbonyl-2-pyridinesulfonamide can be used in combination with 2-chloro-4-ethylamino-6-isopropylamino-1,3,5-triazine, 2-(4-chloro-6-ethylamino-1,3,5-triazin-2-ylamino)-2-methylpropio-nitrile, 2-chloro-2',6'-diethyl-N-(methoxymethyl) acetanilide, 2-chloro-6'-ethyl-N-(2-methoxy-1-methylethyl)aceto-o-toluidide or N-(1-ethylpropyl)-2,6-dinitro-3,4-xylidine. The herbicide of this combination is not noxious for corn and permits substantially completely killing weeds.

Test Example 1

1/1,500 are pots were charged with upland soil, and predetermined amounts of seeds of various test plants were sown. When the test plants had respectively reached the predetermined growth stages (i.e., 2.2- to 3.5-leaf stage for corn ( Zea mays ), 2.0- to 3.5-leaf stage for wheat ( Triticum aestium ), 2.0- to 3.5-leaf stage for common cocklebur, 0.5- to 1.2-leaf stage for tall morningglory 0.5- to 1.2-leaf stage for smartweed, 0.1- to 1.5-leaf stage for prickly sida, 0.1-to 1.5-leaf stage for slender amaranth, and 2.0- to 2.5-leaf stage for barnyard grass), an aqueous disperson was prepared by diluting a wettable powder containing a predetermined amount of each of the test compounds with 5 ℓ/are of water, and 0.2% of an agricultural spreader was added to the aqueous solution. The resultant solution was foliarly applied to the plant with a smallsized sprayer. Twenty-four days after the application, the degree of growth of the plant was visually observed. The weed control was evaluated on a scale of 10 grades in which 10 indicates that the plant was completely killed and 1 indicates no effects, as shown in Table 1 below.

**Table 1**

| Compound No. | Amount of Active Ingredient (g/a) | Weed Control | | | | | | | | Observation Day |
|---|---|---|---|---|---|---|---|---|---|---|
| | | Corn | Wheat | Common Cocklebur | Tall Morning Glory | Prickly Sida | Smart Weed | Slender Amaranth | Barnyard Grass | |
| 1 | 5 | 2 | 10 | 9 | 9 | 9 | 9 | 9 | 9 | 24th day |
| | 1.25 | 1 | 8 | 10 | 8 | 7 | 8 | 10 | 10 | |

Table 1 Contd

| Compound No. | Amount of Active Ingredient (g/a) | Weed Control | | | | | | | | Observation Day |
|---|---|---|---|---|---|---|---|---|---|---|
| | | Corn | Wheat | Common Cocklebur | Tall Morning Glory | Prickly Sida | Smart Weed | Slender Amaranth | Barnyard Grass | |
| 2 | 1.25 | 1 | 10 | 10 | 10 | 6 | 9 | 10 | 10 | 24th day |
| 3 | 1.25 | 1 | 10 | 9 | 9 | 5 | 9 | 10 | 10 | 24th day |

Compounds 1, 2 and 3 in Table 1 above are the compound of the formula (I), the sodium salt thereof and the monomethylamine salt thereof, respectively.

Test Example 2

1/10.000 are pots were respectively charged with upland soil, and seeds of crab-grass and black nightshade were sown. Thereafter, wnen test plants of crab-grass and black nightshade had reached 2- and 0.5-leaf stages, respectively, a compound to be used was weighed such that the content of the active ingredient was a predetermined amount, and the compound was diluted in 5 ℓ water per are, 0.2% of an agricultrual spreader was added to this aqueous solution, and the resultant solution was sprayed with a small-sized sprayer. The rate of growth of crab-grass and black nightshade 23rd days after the application was visually checked, and weed control was evaluated in the same manner as in test example I. The results are shown in Table 2.

Table 2

| Compound No. | Amount of Active Ingredient (g/a) | Weed Control | |
|---|---|---|---|
| | | Crab-grass | Black Nightshade |
| 1 | 5.0 | 10 | 10 |
| | 2.5 | 10 | 10 |

Test Example 3

Two 15 cm long germinated rhizomes of johnsongrass were planted in a 1/3000 are pot and grown in a greenhouse. When the johnsongrass had reached 4- to 5-leaf stage, compounds to be used were weighed such that the content of the active ingredient was a predetermined amount, and the compounds were diluted with 5 ℓ water per are. In addition. 0.2% of an agricultural spreader was added to this aqueous solution, and the resultant solution was sprayed to the leaves and stems of the plants. Thirty-five days after the application, weed control for johnsongrass was visually checked. Weed control was evaluated in the same manner as in test example I, and results are shown in Table 3 below.

Table 3

| Compound No. | Amount of Active Ingredient (g/a) | Weed Control |
|---|---|---|
| 1 | 2.5 | 9 ~ 10 |
| | 1.25 | 9 ~ 10 |
| | 0.625 | 8 ~ 9 |
| | 0.313 | 7 |

Test Example 4

Four germinated tubers of purple nutsedge were planted in a 1/10,000 are pot and grown in a greenhouse. When the purple nutsedge had reached 3- to 4-leaf stage, compounds to be used were weighed such that the content of the active ingredient was a predetermined amount, and the compounds were diluted with 5 ℓ water per are. In addition, 0.2% of an agricultural spreader was added to this aqueous solution, and the resultant solution was sprayed to the leaves of the plants. Fifty-one days after the application, weed control for purple nutsedge was visually checked. Weed control was evaluated in the same manner as in test example 1, and results are shown in Table 4 below.

Table 4

| Compound No. | Amount of Active Ingredient (g/a) | Weed Control |
|---|---|---|
| 1 | 2.5 | 10 |
| | 1.25 | 10 |
| | 0.625 | 10 |
| | 0.313 | 8 |

Formulation preparation examples of herbicidal compositions according to the present invention will be described below.

Formulation Example 1

| | | parts by weight |
|---|---|---|
| (1) | Water-soluble starch | 55 |
| (2) | Sodium lignosulfonate | 5 |
| (3) | Compound No.2 | 40 |

The above ingredients are mixed and pulvarized to form an aqueous solution.

Formulation Example 2

| | | parts by weight |
|---|---|---|
| (1) | Kaoline | 78 |
| (2) | Condensate of sodium naphthalensulfonate and formalin | 2 |
| (3) | Polyoxyethylenealkylallyl-ethersulfate | 5 |
| (4) | Silica micropowder | 15 |

Ingredients (1) to (4) are mixed with the compounds, of the present invention at a mixing ratio of 9 : 1 to prepare a wettable powder.

Formulation Example 3

| | | parts by weight |
|---|---|---|
| (1) | Diatomaceous earth | 63 |
| (2) | Polyoxyethylenealkyl-phenylethersulfate ammonium salt | 5 |
| (3) | Dialkylsulfosuccinate | 2 |
| (4) | Compound No.1 | 30 |

The above ingredients are mixed to form a wettable powder.

In Tables 2 to 4 and Formulation Example 3, Compound No.1 is the compound of the formula (I) and in Formulation Example 1, Compound No.2 is the sodium salt of the compound of the formula (I).

## Claims

**Claims for the following Contracting States : CH, DE, FR, GB, IT, LI, NL**

1. A substituted pyridine sulfonamide compound having the formula:

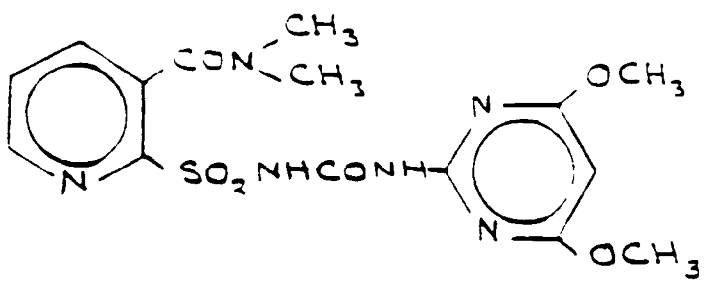

, or a salt thereof.

2. N-[(4,6-dimethoxypyrimid-2-yl)aminocarbonyl]-3-dimethylaminocarbonyl-2-pyridinesulfonamide.

3. Sodium salt of N-[(4,6-dimethoxypyrimid-2-yl)aminocarbonyl]-3-dimethylaminocarbonyl-2-pyridinesulfonamide.

4. A herbicidal composition essentially consisting of a herbicidally effective amount of a compound as claimed in any preceding claim, and an agriculturally acceptable adjuvant.

5. A composition as claimed in claim 4, wherein the mixing ratio of the compound or salt thereof to the agriculturally acceptable adjuvant is 1:99 to 90:10.

6. A composition as claimed in claim 4, which also includes a further herbicide.

7. A composition as claimed in claim 6, wherein said further herbicide is selected from 3,6-dichloro-2-methoxybenzoic acid, 2,5-dichloro-3-aminobenzoic acid, (2,4-dichlorophenoxy)acetic acid, (4-chloro-2- methylphenoxy)acetic acid, 2-chloro-4,6-bis(ethylamino)-1,3,5-triazine, 2-chloro-4-ethylamino-6-isopropylamino-1,3,5-triazine, 2-(4-chloro-6-ethylamino-1,3,5-triazin-2-ylamino)-2-methylpropionitrile, 2-ethylamino-4-isopropylamino-6-methylthio-1,3,5-triazine, 2-chloro-2',6'-diethyl-N-(methoxymethyl)-acetanilide, 2-chloro-6'-ethyl-N-(2-methoxy-1-methylethyl)aceto-o-toluidide, 2-chloro-N-isopropylacetanilide, 2-chloro-N,N-di-2-propenylacetamide, S-ethyl dipropylthiocarbamate, S-ethyl di-isobutylthiocarbamate, S-propyl dipropylthiocarbamate, N-(1-ethylpropyl)-2,6-dinitro-3,4-xylidine, α,α,α-trifluoro-2,6-dinitro-N,N-dipropyl-p-toluidine, 2-(3,5-dichlorophenyl)-2-(2,2,2-trichloroethyl)oxirane, 3-isopropyl-(1H)-benzo-2,1,3-thiadiazin-4-one-2,2-dioxide, and 2-chloro-4-trifluoromethylphenyl-3-ethoxy-4-nitrophenylether.

8. A composition as claimed in claim 6, wherein said further herbicide is selected from 2-chloro-4-ethylamino-6-isopropylamino-1,3,5-triazine, 2-(4-chloro-6-ethylamino-1,3,5-triazin-2-ylamino)-2-methyl-propionitrile, 2-chloro-2',6'-diethyl-N-(methoxymethyl)acetanilide, 2-chloro-6'-ethyl-N-(2-methoxy-1-methylethyl)aceto-o-toluidide and N-(1-ethylpropyl)-2,6-dinitro-3,4-xylidine.

9. A method of killing noxious weeds in a cornfield by applying a composition as claimed in claim 4, 5, 6 7 or 8, in an amount of 0.1 to 100g/are based on the active ingredient.

10. A process for producing a substituted pyridinesulfonamide compound having the formula:

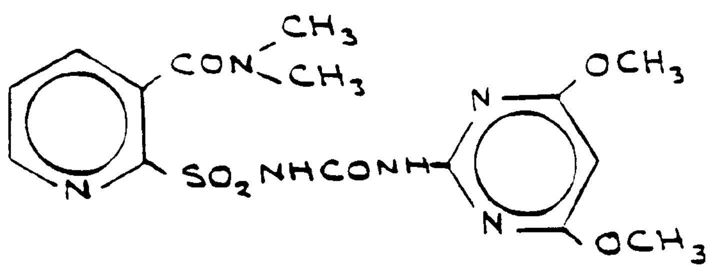

, or a salt thereof, which process comprises reacting a pyridine compound represented by the following general formula:

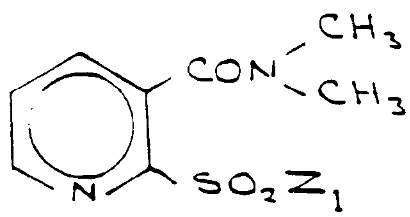

wherein $Z_1$, represents an $-NH_2$ group, an -NCO group, an -NHCOCl group or an $-NHCOOR_5$ group (wherein $R_5$ represents an alkyl group or an aryl group) with a pyrimidine compound represented by the following general formula:

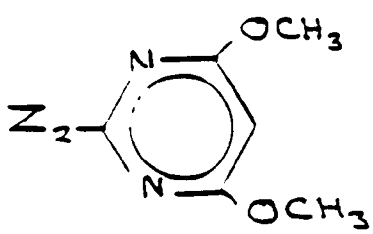

wherein $Z_2$ represents an $-NH_2$ group, an -NCO group, an -NHCOCl group or an $-NHCOOR_5$ group (wherein $R_5$ is as defined above), provided that when $Z_1$ represents the $-NH_2$ group, $Z_2$ represents the -NCO group, the -NHCOCl group or the $-NNCOOR_5$ group, and that when $Z_2$ represents the $-NH_2$ group, $Z_1$, represents the -NCO group, the -NHCOCl group or the $-NHCOOR_5$ group; and if desired, performing a salt formation treatment.

11. A process as claimed in claim 10, wherein $Z_1$ represents an $-NH_2$ group, and $Z_2$ represents an -NCO group, an -NHCOCl group or an $-NHCOOR_5$ group.

12. A process as claimed in claim 10, wherein $Z_1$ represent an -NCO group, an -NHCOCl group or an $-NHCOOR_5$ group, and $Z_2$ represents an $-NH_2$ group.

13. A process according to claim 10, 11 or 12, wherein the temperature of reaction of the pyridine compound

with the pyrimidine compound is -20 to +100°C.

**14.** A pyridine compound as an intermediate represented by the following general formula:

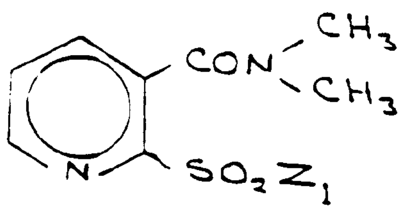

wherein $Z_1$ is an $-NH_2$ group, an -NCO group, an -NHCOCl group or an $-NHCOOR_5$ group (wherein $R_5$ is an alkyl group or an aryl group).

**15.** 2-Aminosulfonyl-N,N-dimethylnicotinamide.

**16.** A compound as claimed in claim 14, wherein $Z_1$ is an $-NHCOOR_5$ group (wherein $R_5$ is a defined in claim 14).

**17.** A compound as claimed in claim 16, wherein $R_5$ is phenyl.

**Claims for the following Contracting States : AT, ES**

**1.** A process for producing a substituted pyridinesulfonamide compound having the formula:

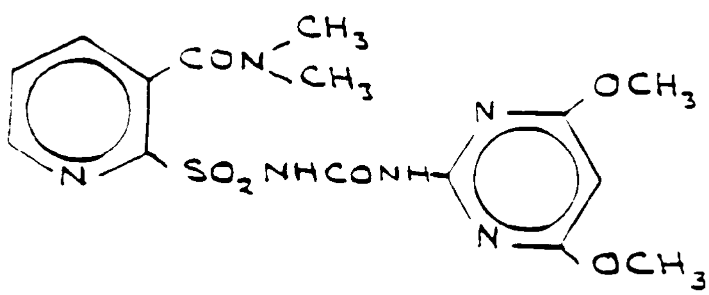

,or a salt thereof, which process comprises reacting a pyridine compound represented by the following general formula:

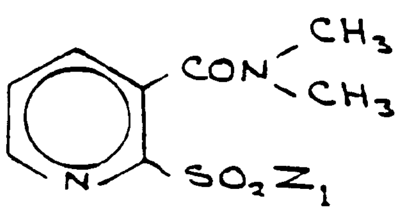

wherein $Z_1$ represents an $-NH_2$ group, an -NCO group, an -NHCOCl group or an $-NHCOOR_5$ group (wherein $R_5$ represents an alkyl group or an aryl group) with a pyrimidine compound represented by the following general formula:

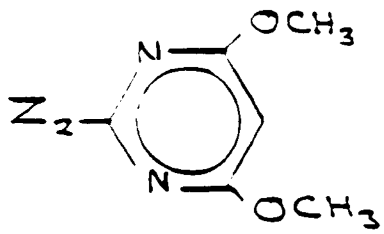

wherein $Z_2$ represents an $-NH_2$ group, an -NCO group, an -NHCOCl group or an $-NHCOOR_5$ group (wherein $R_5$ is as defined above), provided that when $Z_1$ represents an $-NH_2$ group, $Z_2$ represents an -NCO group, an -NHCOCl group or an $-NHCOOR_5$ group, and that when $Z_2$ represents an $-NH_2$ group, $Z_1$ represents an -NCO group, an -NHCOCl group or an $-NHCOOR_5$ group; and if desired, performing a salt formation treatment.

2. A process as claimed in claim 1, wherein $Z_1$ represents an $-NH_2$ group, and $Z_2$ represents an -NCO group, an -NHCOCl group or an $-NHCOOR_5$ group.

3. A process as claimed in claim 1, wherein $Z_1$ represents an -NCO group, an -NHCOCl group or an $-NHCOOR_5$ group, and $Z_2$ represents an $-NH_2$ group.

4. A process according to claim 1, 2 or 3, wherein the temperature of reaction of the pyridine compound with the pyrimidine compound is -20 to +100°C.

5. A process as claimed in claim 1, wherein the compound having the formula defined in claim 1 is produced.

6. A process as claimed in claim 1, wherein the sodium salt is produced.

7. A method of killing noxious weeds in a cornfield by applying 0.1 to 100g/are, based on an active ingredient, of a composition containing, as the active ingredient, a substituted pyridinesulfonamide compound, and an agriculturally acceptable adjuvant, said substituted pyridinesulfonamide compound being represented by the formula:

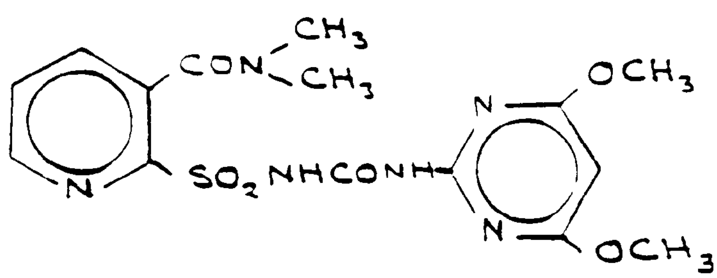

, or a salt thereof.

8. A method as claimed in claim 7, wherein the mixing ratio of the substituted pyridine sulfonamide compound to the agriculturally acceptable adjuvant is 1:99 to 90:10.

9. A method as claimed in claim 7, wherein the composition also contains a further herbicide.

10. A method as claimed in claim 9, wherein the further herbicide is selected from 3,6-dichloro-2-methoxybenzoic acid, 2,5-dichloro-3-aminobenzoic acid, (2,4-dichlorophenoxy)acetic acid, (4-chloro-2-methylphenoxy)acetic acid, 2-chloro-4,6-bis(ethylamino)-1,3,5-triazine, 2-chloro-4-ethylamino-6-isopropylamino-1,3,5-triazine. 2-(4-chloro-6-ethylamino-1,3,5-triazin-2-ylamino)-2-methyl-propionitrile, 2-ethylamino-4-isopropylamino-6-methylthio-1,3, 5-triazine, 2-chloro-2',6'-diethyl-N-(methoxymethyl)acetanilide, 2-chloro-6'-ethyl-N-(2-methoxy-1-methylethyl)aceto-o-toluidide, 2-chloro-N-isopropylacetanilide, 2-chloro-N,N-di-2-propenylacetamide, S-ethyl dipropylthiocarbamate, S-ethyl di-isobutylthiocarbamate, S-propyl dipropylthiocarbamate, N-(1-ethylpropyl)-2,6-dinitro-3,4-xylidine, α,α,α-trifluoro-2,6-dinitro-N,N-dipropyl-p-toluidine, 2-(3,5-dichlorophenyl)-2-(2,2,2-trichloroethyl)oxirane, 3-isopropyl-(1H)-benzo-2,1,3-thiadiazin-4-one-2,2-dioxide, and 2-chloro-4-trifluoromethylphenyl-3-ethoxy-4-nitrophenylether.

**11.** A method as claimed in claim 9, wherein the further herbicide is selected from 2-chloro-4-ethylamino-6-isopropylamino-1,3,5-triazine, 2-(4-chloro-6-ethylamino-1,3,5-triazin-2-ylamino)-2-methyl-propionitrile, 2-chloro-2',6'-diethyl-N-(methoxymethyl)acetanilide, 2-chloro-6'-ethyl-N-(2-methoxy-1-methylethyl)aceto-o-toluidide and N-(1-ethylpropyl)-2,6-dinitro-3,4-xylidine.

**12.** A herbicidal composition of the type defined in any one of claims 7 to 11.

**13.** A method of making a herbicidal composition comprising the step of mixing a substituted pyridinesulfonamide compound as defined in claim 7, and optionally a further herbicide as defined in claim 9 or 10, with an agriculturally acceptable adjuvant.

## Patentansprüche

### Patentansprüche für folgende Vertragsstaaten : CH, DE, FR, GB, IT, LI, NL

**1.** Substituierte Pyridinsulfonamidverbindung der Formel

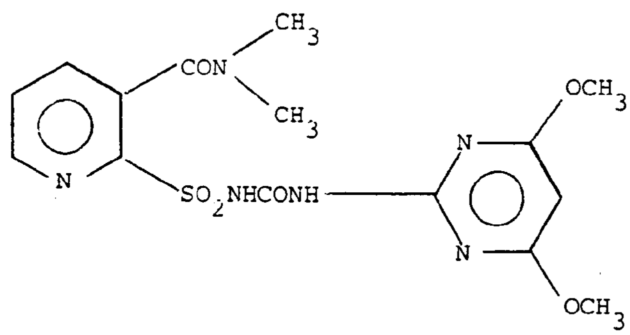

oder ihr Salz.

**2.** N-[(4,6-Dimethoxypyrimid-2-yl)aminocarbonyl]-3-dimethylaminocarbonyl-2-pyridinsulfonamid.

**3.** Natriumsalz von N-[(4,6-Dimethoxypyrimid-2-yl)aminocarbonyl]-3-dimethylaminocarbonyl-2-pyridinsulfonamid.

**4.** Herbizidzusammensetzung, im wesentlichen bestehend aus einer Verbindung in einer herbizid wirksamen Menge nach einem der vorhergehenden Ansprüche und einem landwirtschaftlich annehmbaren Adjuvans.

**5.** Zusammensetzung nach Anspruch 4, worin das Mischungsverhältnis der Verbindung oder ihr Salz zum landwirtschaftlich annehmbaren Adjuvans 1:99 bis 90:10 ist.

**6.** Zusammensetzung nach Anspruch 4, die ein weiteres Herbizid enthält.

**7.** Zusammensetzung nach Anspruch 6, worin das weitere Herbizid ausgewählt ist aus
3,6-Dichloro-2-methoxybenzoesäure,
2,5-Dichloro-3-aminobenzoesäure,
(2,4-Dichlorophenoxy)essigsäure,
(4-Chloro-2-methylphenoxy)essigsäure,
2-Chloro-4,6-bis(ethylamino)-1,3,5-triazin,
2-Chloro-4-ethylamino-6-isopropylamino-1,3,5-triazin,
2-(4-Chloro-6-ethylamino-1,3,5-triazin-2-yl-amino)-2-methyl propionitril,
2-Ethylamino-4-isopropylamino-6-methylthio-1,3,5-triazin,
2-Chloro-2',6'-diethyl-N-(methoxymethyl)acetanilid,

2-Chloro-6'-ethyl-N-(2-methoxy-1-methylethyl)aceto-o-toluidid, 2-Chloro-N-isopropylacetanilid, 2-Chloro-N,N-di-2-propenylacetamid, S-Ethyl-dipropylthiocarbamat, S-Ethyl-diisobutylthiocarbamat, S-Propyl-dipropylthiocarbamat, N-(1-Ethylpropyl)-2,6-dinitro-3,4-xylidin, alpha,alpha,alpha-Trifluoro-2,6-dinitro-N,N-dipropyl-p-toluidin, 2-(3,5-Dichlorophenyl)-2-(2,2,2-trichloroethyl)oxiran, 3-Isopropyl-(1H)-benzo-2,1,3-thiadiazin-4-on-2,2-dioxid, und 2-Chloro-4-trifluoromethylphenyl-3-ethoxy-4-nitrophenylether.

**8.** Zusammensetzung nach Anspruch 6, worin das weitere Herbizid ausgewählt ist aus 2-Chloro-4-ethylamino-6-isopropylamino-1,3,5-triazin, 2-(4-Chloro-6-ethylamino-1,3,5-triazin-2-ylamino)-2-methylpropionitril, 2-Chloro-2',6'-diethyl-N-(methoxymethyl)acetanilid, 2-Chloro-6'-ethyl-N-(2-methoxy-1-methylethyl)aceto-o-toluidid und N-(1-Ethylpropyl)-2,6-dinitro-3,4-xylidin.

**9.** Verfahren zur Vernichtung schädlicher Unkräuter in einem Getreidefeld durch Verwendung einer Zusammensetzung nach Ansprüchen 4, 5, 6, 7 oder 8 in einer Menge von 0,1 bis 100 g/Ar, bezogen auf den Wirkstoff.

**10.** Verfahren zur Herstellung einer substituierten Pyridinsulfonamidverbindung der Formel

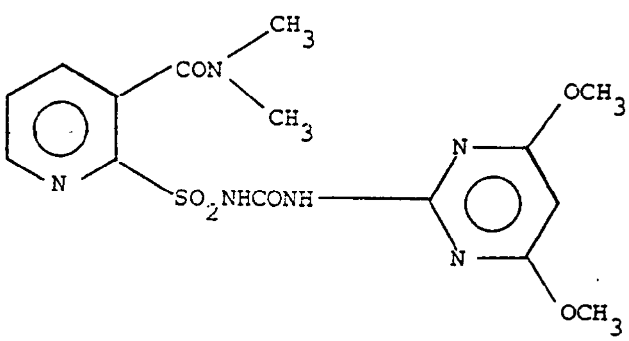

oder ihr Salz, umfassend das Umsetzen einer Pyridinverbindung, dargestellt durch die folgende allgemeine Formel

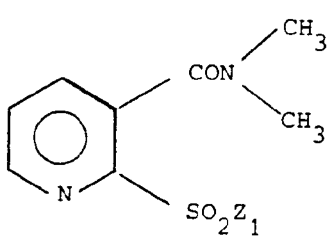

worin $Z_1$ eine $-NH_2$-Gruppe, eine -NCO-Gruppe, eine -NHCOCl-Gruppe oder eine $-NHCOOR_5$-Gruppe ($R_5$ entspricht einer Alkylgruppe oder einer Arylgruppe) darstellt, mit einer Pyrimidinverbindung, dargestellt durch die folgende allgemeine Formel

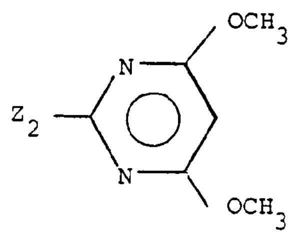

worin $Z_2$ eine $-NH_2$-Gruppe, eine -NCO-Gruppe, eine -NHCOCl-Gruppe oder eine $-NHCOOR_5$-Gruppe ($R_5$ ist wie oben definiert) dargestellt, unter der Voraussetzung, dass, wenn $Z_1$ eine $-NH_2$-Gruppe ist, $Z_2$ die -NCO-Gruppe, die -NHCOCl-Gruppe oder die $-NHCOOR_5$-Gruppe darstellt und dass, wenn $Z_2$ eine $-NH_2$-Gruppe ist, $Z_1$ die -NCO-Gruppe, die -NHCOCl-Gruppe oder die $NHCOOR_5$-Gruppe darstellt, und, falls erwünscht, Herstellung eines Salzes.

**11.** Verfahren nach Anspruch 10, worin $Z_1$ eine $-NH_2$-Gruppe und $Z_2$ eine -NCO-Gruppe, eine -NHCOCl-Gruppe oder eine $-NHCOOR_5$-Gruppe darstellen.

**12.** Verfahren nach Anspruch 10, worin $Z_1$ eine -NCO-Gruppe, eine -NHCOCl-Gruppe oder eine $NHCOOR_5$-Gruppe und $Z_2$ eine $-NH_2$-Gruppe darstellen.

**13.** Verfahren nach den Ansprüchen 10, 11 oder 12, worin die Temperatur bei der Reaktion der Pyridinverbindung mit der Pyrimidinverbindung -20 bis +100°C ist.

**14.** Pyridinverbindung als Zwischenprodukt, dargestellt durch die folgende allgemeine Formel:

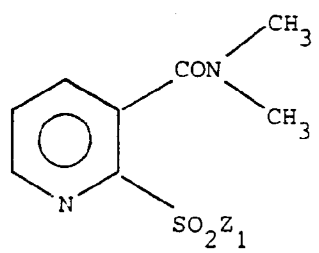

worin $Z_1$ eine $-NH_2$-Gruppe, eine -NCO-Gruppe, eine -NHCOCl-Gruppe oder eine $-NHCOOR_5$-Gruppe ($R_5$ entspricht einer Alkylgruppe oder einer Arylgruppe) darstellt.

**15.** 2-Aminosulfonyl-N,N-dimethylnikotinamid.

**16.** Verbindung nach Anspruch 14, worin $Z_1$ eine $-NHCOOR_5$-Gruppe (worin $R_5$ wie in Anspruch 14 definiert ist) darstellt.

**17.** Verbindung nach Anspruch 16, worin $R_5$ Phenyl ist.

**Patentansprüche für folgende Vertragsstaaten : AT, ES**

**1.** Verfahren zur Herstellung einer substituierten Pyridinsulfonamidverbindung der Formel

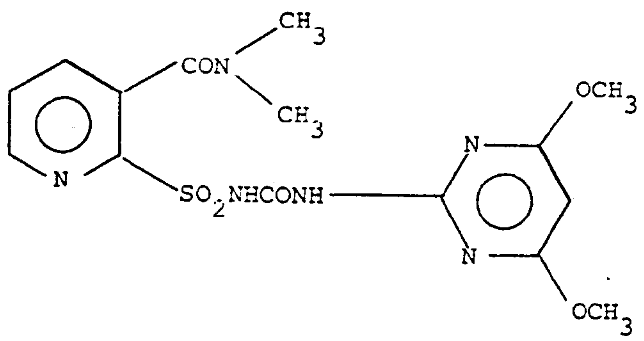

oder ihr Salz, umfassend *das* Umsetzen einer Pyridinverbindung, dargestellt durch die folgende allgemeine Formel

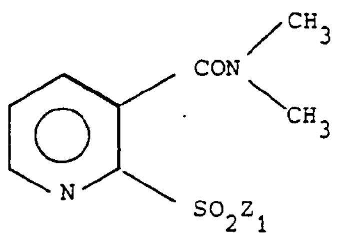

worin $Z_1$ eine $-NH_2$-Gruppe, eine -NCO-Gruppe, eine -NHCOCl-Gruppe oder eine $-NHCOOR_5$-Gruppe ($R_5$ entspricht einer Alkyl- oder einer Arylgruppe) darstellt, mit einer Pyrimidinverbindung, dargestellt durch die folgende allgemeine Formel

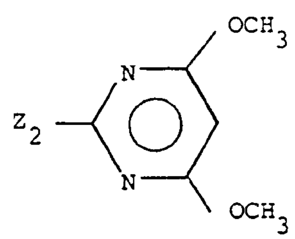

worin $Z_2$ eine $-NH_2$-Gruppe, eine -NCO-Gruppe, eine -NHCOCl-Gruppe oder eine $-NHCOOR_5$-Gruppe ($R_5$ ist wie oben definiert) darstellt, vorausgesetzt, dass, wenn $Z_1$ eine $-NH_2$-Gruppe ist, $Z_2$ eine -NCO-Gruppe, eine -NHCOCl-Gruppe oder eine $NHCOOR_5$-Gruppe darstellt und dass, wenn $Z_2$- eine $-NH_2$-Gruppe ist, $Z_1$ eine -NCO-Gruppe, eine -NHCOCl-Gruppe oder eine $-NHCOOR_5$-Gruppe darstellt, und, falls erwünscht, Herstellung eines Salzes.

2. Verfahren nach Anspruch 1, worin $Z_1$ eine $-NH_2$ -Gruppe und $Z_2$- eine -NCO-Gruppe, eine -NHCOCl-Gruppe oder eine $-NHCOOR_5$-Gruppe darstellt.

3. Verfahren nach Anspruch 1, worin $Z_1$ eine -NCO-Gruppe, eine -NHCOCl-Gruppe oder eine $-NHCOOR_5$-Gruppe und $Z_2$ eine $-NH_2$-Gruppe darstellt.

4. Verfahren nach Ansprüchen 1, 2 oder 3, worin die Reaktionstemperatur der Pyridinverbindung mit der Pyrimidinverbindung -20 bis +100°C ist.

5. Verfahren nach Anspruch 1, worin die Verbindung mit der in Anspruch 1 definierten Formel hergestellt wird.

6. Verfahren nach Anspruch 1, worin das Natriumsalz hergestellt wird.

7. Verfahren zur Vernichtung schädlicher Unkräuter in einem Getreidefeld durch Anwendung von 0,1 bis 100 g/Ar, basierend auf dem aktiven Bestandteil, einer Zusammensetzung, die als aktiven Bestandteil eine

substituierte Pyridinsulfonamidverbindung und ein landwirtschaftlich verwendbares Adjuvans enthält, die substituierte Pyridinsulfonamidverbindung wird durch die Formel

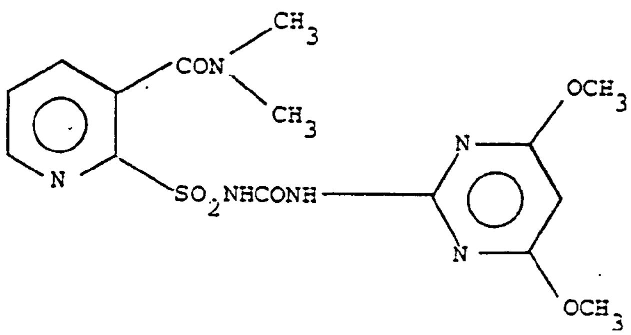

dargestellt, oder ihr Salz.

**8.** Verfahren nach Anspruch 7, worin das Mischungsverhältnis der substituierten Pyridinsulfonamidverbindung zum landwirtschaftlich verwendbaren Adjuvans 1:99 bis 90:10 beträgt.

**9.** Verfahren nach Anspruch 7, worin die Zusammensetzung ein weiteres Herbizid enthält.

**10.** Verfahren nach Anspruch 9, worin das weitere Herbizid ausgewählt ist aus
3,6-Dichloro-2-methoxybenzoesäure,
2,5-Dichloro-3-aminobenzoesäure,
(2,4-Dichlorophenoxy)essigsäure,
(4-Chloro-2-methylphenoxy)essigsäure,
2-Chloro-4,6-bis(ethylamino)-1,3,5-triazin,
2-Chloro-4-ethylamino-6-isopropylamino-1,3,5-triazin,
2-(4-Chloro-6-ethylamino-1, 3,5-triazin-2-yl-amino)-2-methyl propionitril,
2-Ethylamino-4-isopropylamino-6-methylthio-1,3,5-triazin,
2-Chloro-2',6'-diethyl-N-(methoxymethyl)acetanilid,
2-Chloro-6'-ethyl-N-(2-methoxy-1-methylethyl)aceto-o-toluidid, 2-Chloro-N-isopropylacetanilid,
2-Chloro-N,N-di-2-propenylacetamid,
S-Ethyl-dipropylthiocarbamat,
S-Ethyl-diisobutylthiocarbamat,
S-Propyl-dipropylthiocarbamat,
N-(1-Ethylpropyl)-2,6-dinitro-3,4-xylidin,
alpha,alpha,alpha-Trifluoro-2,6-dinitro-N,N-dipropyl-p-toluidin,
2-(3,5-Dichlorophenyl)-2-(2,2,2-trichloroethyl)oxiran,
3-Isopropyl-(1H)-benzo-2,1,3-thiadiazin-4-on-2,2-dioxid,
2-Chloro-4-trifluoromethylphenyl-3-ethoxy-4-nitrophenylether.

**11.** Verfahren nach Anspruch 9, worin das weitere Herbizid ausgewählt ist aus
2-Chloro-4-ethylamino-6-isopropylamino-1,3,5-triazin,
2-(4-Chloro-6-ethylamino-1,3,5-triazin-2-ylamino)-2-methylpropionitril,
2-Chloro-2',6'-diethyl-N-(methoxymethyl)acetanilid,
2-Chloro-6'-ethyl-N-(2-methoxy-1-methylethyl)aceto-o-toluidid und N-(1-Ethylpropyl)-2,6-dinitro-3,4-xylidin.

**12.** Herbizidzusammensetzung vom Typ, wie in einem der Ansprüche 7 bis 11 definiert.

**13.** Verfahren zur Herstellung einer Herbizidzusammensetzung, umfassend den Schritt: Mischen einer substituierten Pyridinsulfonamidverbindung, wie in Anspruch 7 definiert, und wahlweise ein weiteres Herbizid, wie in Ansprüchen 9 oder 10 definiert, mit einem landwirtschaftlich annehmbaren Adjuvans.

## Revendications

### Revendications pour les Etats contractants suivants : CH, DE, FR, GB, IT, LI, NL

1. Un composé pyridine sulfonamide substitué ayant la formule:

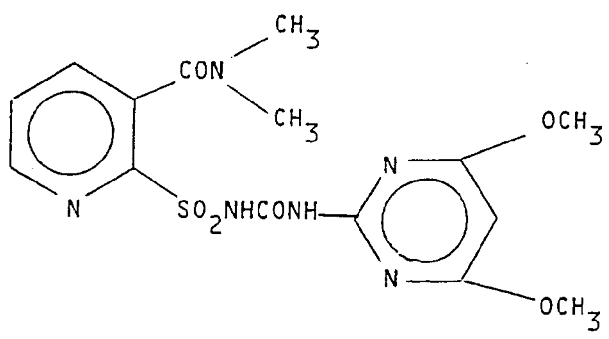

ou un sel de ce composé.

2. N--[(4,6-diméthoxypyrimide-2-yl)aminocarbonyl]-3-diméthylaminocarbonyl-2-pyridine sulfonamide.

3. Sel sodique du N-[(4,6-diméthoxypyrimide-2-yl)aminocarbonyl]-3-diméthylaminocarbonyl-2-pyridine sulfonamide.

4. Une composition herbicide constituée essentiellement d'une quantité herbicide efficace d'un composé selon l'une quelconque des revendications précédentes et d'un adjudant acceptable en agriculture.

5. Une composition selon la revendication 4, selon laquelle le rapport de mélange du composé ou de son sel à l'adjudant acceptable en agriculture est de 1: 99 à 90 : 10.

6. Une composition selon la revendication 4 qui inclut également un autre herbicide.

7. Une composition selon, la revendication 6, selon laquelle cet autre herbicide est choisi parmi l'acide 3,6-dichloro-2-méthoxybenzoïque, l'acide 2,5-dichloro-3-aminobenzoïque, l'acide (2,4-dichlorophénoxy)acétique, l'acide (4-chloro--2--méthylphénoxy)acétique, la 2--chloro--4,6-bis(éthylamino)-1,3,5-triazine, la 2-chloro-4-éthylamino-6-isopropylamino-1,3,5-triazine, le 2-(4-chloro--6-éthylamino--1,3,5--triazine--2-ylamino)-2-méthylpropionitrile, la 2--éthylamino--4--isopropylamino-6-méthylthio-1,3,5-triazine, le 2-chloro-2',6'--diéthyl--N--(méthoxyméthyl)acétanilide, le 2--chloro--6'-éthyl-N-(2-méthoxy-1-méthyléthyl)acéto-o-toluidide, le 2-chloro-N-isopropylacétanilide, le 2-chloro-N,N-di-2-propénylacétamide, le dipropylthiocarbamate de S-éthyle, le di-isobutylthiocarbamate de S-éthyle, le dipropylthiocarbamate de S-propyle, la N-(1-éthylpropyl)-2,6-dinitro-3,4-xylidine, l'α,α,α-trifluoro-2,6-dinitro-N,N-dipropyl-p-toluidine, le 2-(3,5-dichlorophényl)-2-(2,2,2-trichloroéthyl)-oxyranne, le 2,2-dioxyde de 3-isopropyl-(1H)-benzo-2,1,3-thiadiazine-4-one, et l'oxyde de 2-chloro-4-trifluorométhylphényle et de 3-éthoxy-4-nitrophényle.

8. Une composition selon la revendication 6, qui contient également un autre herbicide choisi parmi la 2-chloro-4-éthylamino-6-isopropylamino-1,3,5-triazine, le 2--(4--chloro-6-éthylamino-1,3,5-triazine-2-ylamino)-2-méthyl-propionitrile, le 2-chloro-2',6'-diéthyl-N-(méthoxyméthyl)acétanilide, le 2-chloro-6'-éthyl-N-(2-méthoxy-1--méthyléthyl)acéto--o--toluidide et la N-(1-éthylpropyl)-2,6-dinitro-3,4-xylidine.

9. Une méthode pour détruire les mauvaises herbes nocives dans un champ de maïs par application d'une composition selon la revendication 4, 5, 6, 7 ou 8, en quantité de 0,1 à 100 g/are, basée sur l'ingrédient actif.

10. Un procédé de production d'un composé pyridine sulfonamide ayant la formule:

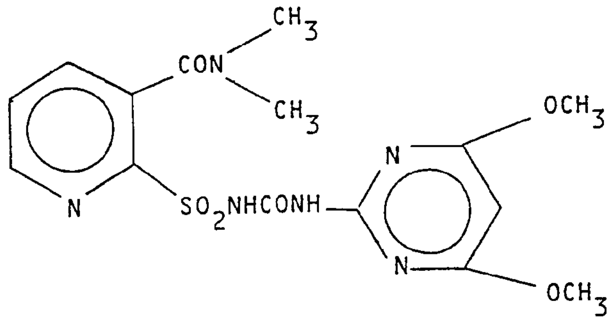

ou d'un sel dudit composé, ledit procédé comprenant la réaction d'un composé pyridine représenté par la formule générale suivante :

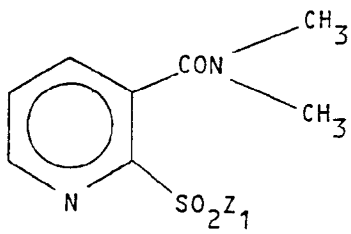

dans laquelle $Z_1$ représente un groupe $-NH_2$, -NCO, -NHCOCl ou $-NHCOOR_5$ (dans lequel $R_5$ représente un groupe alkyle ou aryle) avec un composé pyrimidine représenté par la formule générale suivante :

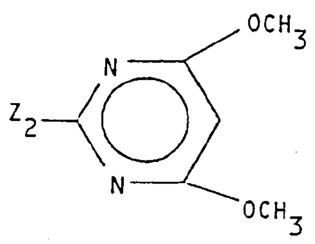

dans laquelle $Z_2$ représente un groupe $-NH_2$, -NCO, -NHCOCl ou $-NHCOOR_5$ (dans lequel $R_5$ est comme défini ci-dessus), à la condition que, lorsque $Z_1$ représente un groupe $-NH_2$ , $Z_2$ représente un groupe -NCO, -NHCOCl ou $-NHCOOR_5$, et, lorsque $Z_2$ représente un groupe $-NH_2$, $Z_1$ représente un groupe -NCO, -NHCOCl ou $-NHCOOR_5$ ; et, si on le désire, on procède à un traitement pour former un sel.

**11.** Un procédé selon la revendication 10, selon lequel $Z_1$, représente un groupe $-NH_2$, et $Z_2$ représente un groupe -NCO, -NHCOCl, ou $-NHCOOR_5$.

**12.** Un procédé selon la revendication 10, selon lequel $Z_1$ représente un groupe -NCO, -NHCOCl, ou $-NHCOOR_5$, et $Z_2$ représente un groupe $-NH_2$.

**13.** Un procédé selon la revendication 10, 11, ou 12, selon lequel la température de réaction du composé pyridine avec le composé pyrimidine est de -20 à +100°C.

**14.** Un composé pyridine en tant qu'intermédiaire représenté par la formule générale suivante :

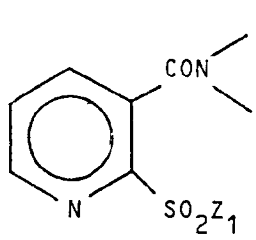

dans laquelle $Z_1$ est un groupe $-NH_2$, -NCO, -NHCOCl ou $-NHCOOR_5$ (dans lequel $R_5$ est un groupe alkyle ou aryle).

**15.** 2-aminosulfonyl-N,N-diméthylnicotinamide.

**16.** Un composé selon la revendication 14, selon lequel $Z_1$ est un groupe $-NHCOOR_5$ (dans lequel $R_5$ est comme défini dans la revendication 14).

**17.** Un composé selon la revendication 16, selon lequel $R_5$ est un groupe phényle.

**Revendications pour les Etats contractants suivants : AT, ES**

**1.** Un procédé de production d'un composé pyridinesulfonamide substitué ayant la formule :

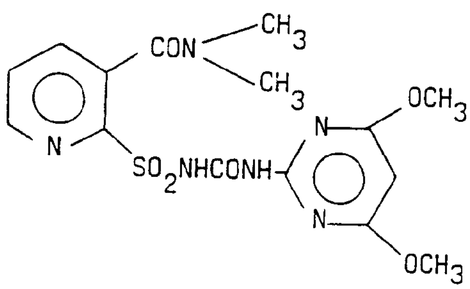

ou d'un sel dudit composé, ledit procédé comprenant la réaction d'un composé pyridine représenté par la formule générale suivante :

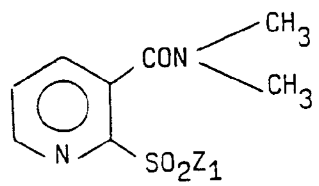

dans laquelle $Z_1$ représente un groupe $-NH_2$, -NCO, -NHCOCl ou $-NHCOOR_5$ (dans lequel $R_5$ représente un groupe alkyle ou un groupe aryle) avec un composé pyrimidine représenté par la formule générale suivante :

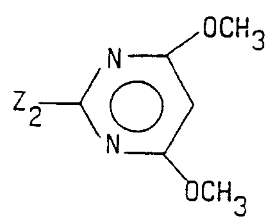

dans laquelle $Z_2$ représente un groupe $-NH_2$, -NCO, -NHCOCl ou $-NHCOOR_5$ (dans lequel $R_5$ est comme défini ci-dessus), à la condition que lorsque $Z_1$ représente un groupe $-NH_2$, $Z_2$ représente un groupe -NCO,

-NHCOCl ou -NHCOOR$_5$, et lorsque $Z_2$ représente un groupe -NH$_2$, $Z_1$ représente un groupe -NCO, -NHCOCl ou -NHCOOR$_5$ ; et si on le désire, on procède à un traitement pour former un sel.

2. Un procédé selon la revendication 1, selon laquelle $Z_1$ représente un groupe -NH$_2$ et $Z_2$ représente un groupe -NCO, un groupe -NHCOCl ou un groupe -NHCOOR$_5$.

3. Un procédé selon la revendication 1, selon laquelle $Z_1$ représente un groupe -NCO et un groupe -NHCOCl ou un groupe -NHCOOR$_5$ et $Z_2$ représente un groupe -NH$_2$.

4. Un procédé selon la revendication 1, 2 ou 3, selon laquelle la température de réaction du composé pyridine avec le composé pyrimidine est de -20 à +100°C.

5. Un procédé selon la revendication 1, selon laquelle le composé ayant la formule définie dans la revendication 1 est produit.

6. Un procédé selon la revendication 1, selon laquelle le sel de sodium est produit.

7. Une méthode pour détruire les mauvaises herbes nuisibles dans un champ de maïs par application de 0,1 à 100 g/a basé sur l'ingrédient actif, d'une composition contenant, comme ingrédient actif, un composé pyridinesulfonamide substitué, et un adjuvant acceptable en agriculture, ledit composé pyridinesulfonamide substitué étant représenté par la formule :

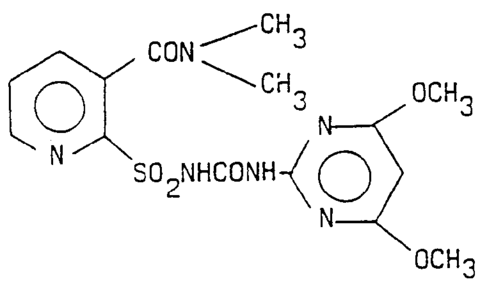

ou un sel dudit composé.

8. Une méthode selon la revendication 7, selon laquelle le rapport de mélangeage du composé pyridinesulfonamide substitué à l'adjuvant acceptable en agriculture est de 1 : 99 à 90 : 10.

9. Une méthode selon la revendication 7, selon laquelle la composition contient également un autre herbicide.

10. Une méthode selon la revendication 9, selon laquelle l'autre herbicide est choisi parmi les suivants : acide 3,6-dichloro-2-méthoxybenzoïque, acide 2,5-dichloro-3-aminobenzoïque, acide (2,4-dichlorophénoxy)acétique, acide (4-chloro-2-méthylphénoxy)acétique, acide 2-chloro-4,6-bis(éthylamino)-1,3,5-triazine, 2-chloro-4-éthylamino-6-isopropylamino-1,3,5-triazine, 2-(4-chloro-6-éthylamino-1,3,5-triazine-2-ylamino)-2-méthylpropionitrile, 2-éthylamino-4-isopropylamino-6-méthylthio-1,3,5-triazine, 2-chloro-2',6'-diéthyl-N-(méthoxyméthyl)acétanilide, 2-chloro-6'-éthyl-N-(2-méthoxy-1-méthyléthyl)acéto-o-toluidide, 2-chloro-N-isopropylacétanilide, 2-chloro-N,N-di-2-propénylacétamide, dipropylthiocarbamate de S-éthyle, diisobutylthiocarbamate de S-éthyle, dipropylthiocarbamate de S-propyle, N-(1-éthylpropyl)-2,6-dinitro-3,4-xylidine, $\alpha,\alpha,\alpha$-trifluoro-2,6-dinitro-N,N-dipropyl-p-toluidine. 2-(3,5-dichlorophényl)-2-(2,2,2-trichloroéthyl)oxiranne, 3-isopropyl-(1H)-benzo-2,1,3-thiadiazine-4-one-2,2-dioxyde et 2-chloro-4-trifluorométhylphényl-3-éthoxy-4-nitrophényléther.

11. Une méthode selon la revendication 9, selon laquelle l'autre herbicide est choisi parmi les suivants : 2-chloro-4-éthylamino-6-isopropylamino-1,3,5-triazine, 2-(4-chloro-6-éthylamino-1,3,5-triazine-2-ylamino)-2-méthylpropionitrile, 2-chloro-2',6'-diéthyl-N-(méthoxyméthyl)acétanidine, 2-chloro-6'-éthyl-N-(2-méthoxy-1-méthyléthyl)acéto-o-toluidide et N-(1-éthylpropyl)-2,6-dinitro-3,4-xylidine.

12. Une composition herbicide du type défini dans l'une quelconque des revendications 7 à 11.

13. Une méthode de fabrication d'une composition herbicide comprenant l'étape de mélangeage d'un composé pyridinesulfonamide substitué tel que défini dans la revendication 7, et éventuellement un autre herbicide tel que défini dans la revendication 9 ou 10, avec un adjuvant acceptable en agriculture.